(19) Europäisches Patentamt
European Patent Office
Office européen des brevets

(11) Publication number: 0 350 946 A2

(12) EUROPEAN PATENT APPLICATION

(21) Application number: 89112952.0

(51) Int. Cl.4: C07D 277/56 , A01N 43/78

(22) Date of filing: 14.07.89

Claims for the following Contracting State: ES.

(30) Priority: 15.07.88 JP 177750/88
09.05.89 JP 116875/89

(43) Date of publication of application:
17.01.90 Bulletin 90/03

(84) Designated Contracting States:
DE ES FR GB IT NL

(71) Applicant: SUMITOMO CHEMICAL COMPANY, LIMITED
5-33 Kitahama 4-chome Chuo-ku
Osaka-shi Osaka(JP)

(72) Inventor: Kusaba, Tomoyuki
2-10-4-420, Sonehigashi-machi
Toyonaka-shi Osaka-fu(JP)
Inventor: Tamaki, Masahiro
2-14-7, Mefu
Takarazuka-shi Hyogo-ken(JP)
Inventor: Uematsu, Tamon
1-13-13, Sakuragaokahigashi-machi Nishi-ku
Kobe-shi Hyogo-ken(JP)

(74) Representative: Vossius & Partner
Siebertstrasse 4 P.O. Box 86 07 67
D-8000 München 86(DE)

(54) **Amide derivatives, and their production and agricultural and horticultural fungicides containing them.**

(57) An amide compound of the formula:

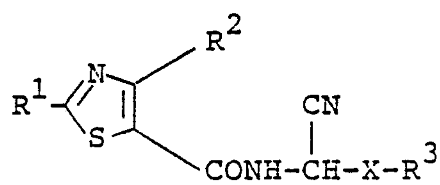

wherein $R^1$ and $R^2$ are, the same or different, each a a lower alkyl group, $R^3$ is a lower alkyl group, a halo(lower)alkyl group, a cyano(lower)alkyl group or a lower alkynyl group and X is an oxygen atom or a sulfur atom, which is useful as a fungicide.

EP 0 350 946 A2

## AMIDE DERIVATIVES, AND THEIR PRODUCTION AND AGRICULTURAL AND HORTICULTURAL FUNGICIDES CONTAINING THEM

The present invention relates to novel amide derivatives, and their production and agricultural and horticultural fungicides containing them.

As the prevailing agricultural fungicides for controlling plant diseases caused by phytopathogenic fungi such as Pythiaceae and Peronosporaceae, there are known captan, captafol, dithiocarbamates, etc. However, almost all of them show a preventive effect, and a curative effect is hardly produced thereby. Such fungicidal activity is not sufficient to control plant diseases when applied after germination of phytopathogenic fungi. This is disadvantageous, because application of fungicides after slight germination of phytopathogenic fungi is sometimes required depending on the circumstances. Therefore, there is always a strong demand for a fungicide exerting a high preventive effect and an excellent systemic activity so as to produce a curative effect. This is particularly true for controlling plant diseases showing rapid development of symptoms, for instance, caused by Peronosporaceae. Recently, there was provided metalaxyl (i.e. N-(2,6-dimethylphenyl)-N-(methoxyacetyl)alanine methyl ester) having an excellent systemic activity and showing a good curative effect. Since, however, organisms resistant to metalaxyl appeared shortly after its commercialization, its curative effect could not be fully appreciated. Under the above circumstances, the appearance of a new fungicide having an excellent systemic activity and showing a prominent curative effect, particularly of the one effectively applicable to downy mildew of grapes, has been highly demanded in the world.

As a result of the extensive study, it has now been found that amide compounds of the formula:

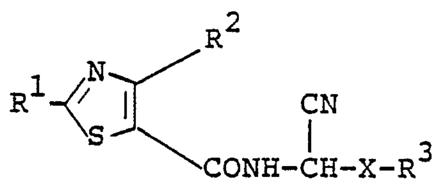

(I)

wherein $R^1$ and $R^2$ are, the same or different, each a a lower alkyl group, $R^3$ is a lower alkyl group, a halo(lower)alkyl group, a cyano(lower)alkyl group or a lower alkynyl group and X is an oxygen atom or a sulfur atom have a strong antifungal activity with an excellent systemic activity and a curative effect without phytotoxicity.

Among the amide compounds (I), preferred are those of the formula:

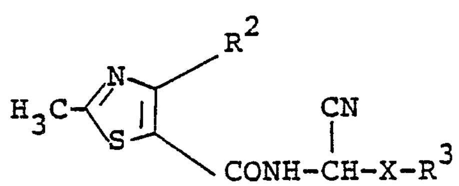

(I-1)

wherein $R^2$ is a methyl group or an ethyl group and $R^3$ is a $C_1$-$C_3$ alkyl group, a halo($C_2$-$C_3$)alkyl group, a cyano($C_1$-$C_3$) alkyl group or a $C_3$-$C_5$ alkynyl group and X is an oxygen atom or a sulfur atom.

More preferred are those of the formula:

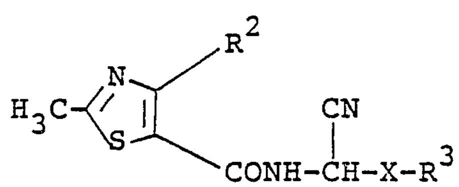

(I-2)

wherein $R^2$ is a methyl group or an ethyl group and $R^3$ is a $C_1$-$C_3$ alkyl group and X is an oxygen atom or a sulfur atom.

Particularly preferred are those of the formula:

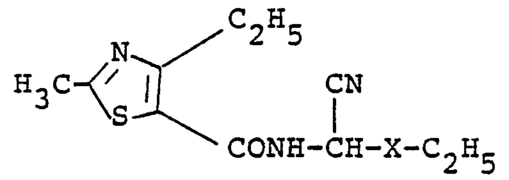

(I-3)

wherein X is an oxygen atom or a sulfur atom.

Hitherto, there have been known a great number of amide compounds which are useful as fungicides and/or herbicides. Among them, similar to the amide compounds (I) in chemical structure are the compounds of the formula (I) wherein the thiazole ring is substituted with a substituted benzene, furan, thiophene, benzofuran or pyridine ring as disclosed in EP-A-0059536 and EP-A-0268892, the compounds of the formula (I) wherein the thiazole ring is substituted with a substituted benzene ring as disclosed in EP-A-0171768 and JP-A-255759/1985, etc. However, these known compounds are not sufficient in efficacy on plant diseases, particularly diseases caused by phytopathogenic fungi such as Peronosporaceae (e.g. downy mildew, late bright) and also in systemic activity. In addition, their phytotoxicity is considerably strong. It may be thus stated that the remarkable curative effect of the amide compounds (I), especially compound (I-3), on plant diseases caused by phytopathogenic fungi without producing any material phytotoxicity is quite unexpected from above mentioned known patents and literatures.

The amide compounds (I) may be produced, for instance, by reacting a haloacetonitrile of the formula:

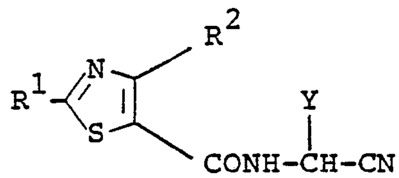

(II)

wherein $R^1$ and $R^2$ are each as defined above and Y is a halogen atom (e.g. chlorine, bromine) with a compound of the formula:

$R^3$-X-H (III)

wherein $R^3$ and X are each as defined above in their reactive forms.

The above reaction is usually performed successively to the reaction of an acetonitrile of the formula:

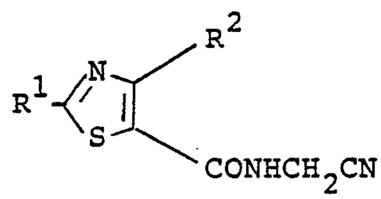

(IV)

wherein $R^1$ and $R^2$ are each as defined above with a halogenating agent for production of the haloacetonitrile (II), because of the relative unstability of the haloacetonitrile (II).

Namely, in the initial step, the acetonitrile (IV) is reacted with a halogenating agent (e.g. chlorine, bromine, pyridinium perbromide) in the presence of a Lewis acid (e.g. hydrochloric acid, hydrobromic acid, phosphorous tribromide), normally at a temperature of about 10 to 50 °C instantaneously or within about 3 hours. The reaction is preferably carried out in a solvent such as a halogenated hydrocarbon (e.g. chloroform, carbon tetrachloride, dichloroethane) or an ester (e.g. ethyl acetate, butyl acetate). The amount of the halogenating agent may be usually from about 1 to 3 equivalents to one equivalent of the acetonitrile (IV). The haloacetonitrile (II) thus produced is, in general, not sufficiently stable and subjected to the reaction in the subsequent step without isolation.

In the subsequent step, the haloacetonitrile (II) is reacted with the compound (III) at a temperature of about -30 to 50 °C, preferably about 0 °C to room temperature, for a period of about 30 minutes to 24 hours, preferably about 1 to 8 hours, normally in the presence of a base. Examples of the base are tertiary amines (e.g. pyridine, triethylamine, N,N-dimethylaniline, tributylamine, N-methylmorpholine), inorganic bases (e.g. sodium hydroxide, potassium hydroxide, calcium carbonate), etc. The compound (III) may be used in an amount of about 1 to 100 equivalents to one equivalent of the haloacetonitrile (II). The base may

be used in an amount of about 1 to 50 equivalents to one equivalent of the haloacetonitrile (II).

After completion of the reaction, the reaction mixture may be subjected to post-treatment in a per se conventional manner, for instance, washing with water, separating and concentrating the organic layer and optionally purifying the resulting product. For purification, chromatography or recrystallization may be adopted.

The starting acetonitrile (IV) is obtainable by various procedures as set forth below.

Procedure (A):-

According to this procedure, a thiazolecarboxylic acid halide of the formula:

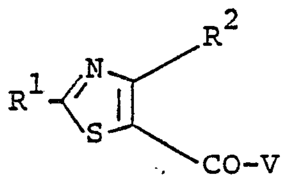

(V)

wherein $R^1$ and $R^2$ are each as defined above and V is a halogen atom (e.g. chlorine, bromine) is reacted with aminoacetonitrile of the formula: $H_2NCH_2CN$ or its salt to give the acetonitrile (IV).

In general, the reaction is carried out at a temperature of about -30 to 50 °C, preferably about 0 °C to room temperature, for a period of about 30 minutes to 24 hours, preferably about 1 to 8 hours, normally in the presence of a base. Examples of the base are tertiary amines (e.g. pyridine, triethylamine, N,N-dimethylaniline, tributylamine, N-methylmorpholine), inorganic bases (e.g. sodium hydroxide, potassium hydroxide, calcium carbonate), etc. The use of a solvent is usually favorable, and examples of the solvent are aliphatic hydrocarbons (e.g. hexane, heptane, ligroin, petroleum ether), aromatic hydrocarbons (e.g. benzene, toluene, xylene), halogenated hydrocarbons (e.g. chloroform, carbon tetrachloride, dichloroethane, chlorobenzene, dichlorobenzene), ethers (e.g. diethyl ether, diisopropyl ether, dioxane, tetrahydrofuran, ethylene glycol dimethyl ether), ketones (e.g. acetone, methyl ethyl ketone, methyl isobutyl ketone, isophorone, cyclohexanone), esters (e.g. ethyl formate, ethyl acetate, butyl acetate, diethyl carbonate), nitro compounds (e.g. nitromethane, nitrobenzene), nitriles (e.g. acetonitrile, isobutylnitrile), acid amides (e.g. formamide, N,N-dimethylformamide, N,N-dimethylacetamide), sulfur compounds (e.g. dimethylsulfoxide, sulfolane), etc. These solvents may be used alone or in combination. Aminoacetonitrile or its salt and the base may be employed respectively in amounts of about 1 to 2 equivalents and of about 1 to 2 equivalents to one equivalent of the thiazolecarboxylic acid halide (V).

After completion of the reaction, the reaction mixture may be subjected to post-treatment in a per se conventional manner, for instance, by washing with water, separating and concentrating the organic layer and optionally purifying the resulting product. For purification, chromatography or recrystallization may be adopted.

Procedure (B):-

According to this procedure, a thiazolecarboxylic acid of the formula:

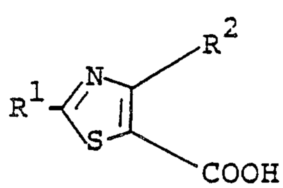

(VI)

wherein $R^1$ and $R^2$ are each as defined above is reacted with a diimidazole of the formula:

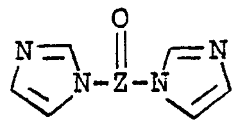

(VII)

wherein Z is a carbon atom or a sulfur atom to give an acylimidazole of the formula:

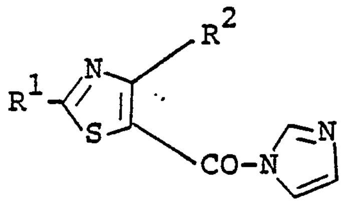

(VIII)

wherein $R^1$ and $R^2$ are each as defined above, and then the acylimidazole (VIII) is reacted with aminoacetonitrile or its salt to give the acetonitrile (IV).

The reactions in the initial step and the sub sequent step are normally performed successively without isolation of the acylimidazole (VIII), because it is in general not sufficiently stable. Those reactions may be carried out at a temperature of about -30 to 50 °C, preferably about 0 °C to room temperature, for a period of about 30 minutes to 24 hours, preferably about 1 to 8 hours. The use of a solvent is normally preferred, and examples of the solvent are those as exemplified with respect to Procedure (A). The proportion of the thiazolecarboxylic acid (VI), the diimidazole (VII) and aminoacetonitrile or its salt is usually about 1 : 1 : 1 - 2 in equivalent.

After completion of the reaction, the reaction mixture may be subjected to post-treatment in a per se conventional manner, for instance, by washing with water, separating and concentrating the organic layer and optionally purifying the resultant product. For purification, chromatography or recrystallization may be adopted.

Procedure (C):-

According to this procedure, an acylacetonitrile of the formula:

$R^2-COCH_2CONHCH_2CN$ (IX)

wherein $R^2$ is as defined above is reacted with a thioacetamide of the formula:

$R^1-\overset{\overset{S}{\|}}{C}-NH_2$ (X)

wherein $R^1$ is as defined above in the presence of a halo genating agent (e.g. chlorine, bromine, sulfuryl chloride) to give the acetonitrile (IV).

The reaction may be carried out at a temperature of about -30 to 100 °C, preferably about 0 to 80 °C, for a period of about 30 minutes to 24 hours, preferably about 1 to 8 hours. The reaction is favorably effected in a solvent chosen from aliphatic hydrocarbons (e.g. hexane, heptane, ligroin, petroleum ether), aromatic hydrocarbons (e.g. benzene, toluene, xylene), halogenated hydrocarbons (e.g. chloroform, carbon tetrachloride, dichloroethane, chlorobenzene, dichlorobenzene), ethers (e.g. diethyl ether, diisopropyl ether, dioxane, tetrahydrofuran, ethylene glycol dimethyl ether), ketones (e.g. acetone, methyl ethyl ketone, methyl isobutyl ketone, isophorone, cyclohexanone), esters (e.g. ethyl formate, ethyl acetate, butyl acetate, diethyl carbonate), nitro compounds (e.g. nitromethane, nitrobenzene), nitriles (e.g. acetonitrile, isobutylnitrile), acid amides (e.g. formamide, N,N-dimethylformamide, N,N-dimethylacetamide), sulfur compounds (e.g. dimethylsulfoxide, sulfolane), etc. These solvents may be used solely or in combination. The thioacetamide (X) and the halogenating agent may be employed respectively in amounts of about 1 to 2 equivalents and of about 1 to 2 equivalents to one equivalent of the acylacetonitrile (IX).

In the above procedures, some of the thiazolecarboxylic acid (VI) and the thiazolecarboxylic acid halide (V) are known, and some others may be produced by per se conventional procedures. For instance, 2,4-disubstituted thiazole-5-carboxylic acids are disclosed in Ann., 250, 257 (1889), and 2,4-disubstituted thiazole-5-carbxylic acid chlorides are disclosed in J.Chem.Soc., 601 (1945). Likewise, Angew. Chem., 73, 26 (1961) discloses the preparation method of the the diimidazole (VII).

The amide compounds (I) of the invention have one asymmetric carbon atom in their molecules, and

therefore there are optical isomers. These are also included in the scope of the invention.

Practical embodiments for preparation of the amide compound (I) and the acetonitrile (IV) are shown in the following Examples.

Example 1

To a solution of 2-(2,4-dimethylthiazole-5-carboxamino)acetonitrile (2.0 g; 10 mmol) in ethyl acetate (40 ml), anhydrous magnesium sulfate (5 g) and bromine (3.2 g; 20 mmol) were added while stirring at room temperature. To the resultant suspension was added dropwise 47 % of hydrobromic acid (1.7 g; 10 mmol) at room temperature under vigorous stirring, and stirring was continued for 1 hour. A mixture of triethylamine (5.1 g; 50 mmol) and ethanol (0.51 g; 11 mmol) was dropwise added thereto under ice-cooling with stirring, and stirring was continued at room temperature for 1 hour. The reaction mixture was diluted with ethyl acetate, washed with water twice, dried over anhydrous magnesium sulfate and concentrated under reduced pressure to give a crude oil. The oil was purified by column chromatography (eluent, n-hexane : ethyl acetate = 2 : 1) to give 1.45 g of 2-ethoxy-2-(2,4-dimethylthiazole-5-carboxamino)acetonitrile (Compound No. 2) as a viscous oil. Yield, 60 %.

Example 2

To a solution of 2-(2,4-dimethylthiazole-5-carboxamino)acetonitrile (4.0 g; 20 mmol) in ethyl acetate (70 ml), anhydrous magnesium sulfate (10 g) and bromine (6.5 g; 40 mmol) were added while stirring at room temperature. To the resultant suspension was added dropwise 47 % of hydrobromic acid (3.4 g; 20 mmol) at room temperature under vigorous stirring, and stirring was continued for 1 hour. A mixture of triethylamine (10 g; 100mmol) and 1-butyn-3-ol (2.1 g; 11 mmol) was dropwise added thereto under ice-cooling with stirring, and stirring was continued at room temperature for 1 hour. The reaction mixture was diluted with ethyl acetate, washed with water twice, dried over anhydrous magnesium sulfate and concentrated under reduced pressure to give a crude oil. The oil was purified by column chromatography (eluent, n-hexane : ethyl acetate = 2 : 1) to give 2.8 g of 2-(butynyl-3-oxy)-2-(2,4-dimethylthiazole-5-carboxamino)acetonitrile (Compound No. 4) as a viscous oil (diastereo mixture). Yield, 53 %.

Example 3

To a solution of 2-(4-ethyl-2-methylthiazole-5-carboxamino)acetonitrile (2.1 g; 10 mmol) in ethyl acetate (40 ml), anhydrous magnesium sulfate (5 g) and bromine (3.2 g; 20 mmol) were added while stirring at room temperature. To the resultant suspension was added dropwise 47 % of hydrobromic acid (1.7 g; 10 mmol) at room temperature under vigorous stirring, and stirring was continued for 1 hour. A mixture of triethylamine (5.1 g; 50 mmol) and ethanol (5 g; 108 mmol) was dropwise added thereto under ice-cooling with stirring, and stirring was continued at room temperature for 1 hour. The reaction mixture was diluted with ethyl acetate, washed with water twice, dried over anhydrous magnesium sulfate and concentrated under reduced pressure to give a crude oil. The oil was purified by column chromatography (eluent, n-hexane : ethyl acetate = 2 : 1) to give 1.2 g of 2-ethoxy-2-(4-ethyl-2-methylthiazole-5-carboxamino)-acetonitrile (Compound No. 6). m.p., 126 - 127 °C. Yield, 48 %.

Example 4

To a solution of 2-(4-ethyl-2-methylthiazole-5-carboxamino)acetonitrile (2.1 g; 10 mmol) in ethyl acetate (40 ml), anhydrous magnesium sulfate (5 g) and bromine (3.2 g; 20 mmol) were added while stirring at room temperature. To the resultant suspension was added dropwise 47 % of hydrobromic acid (1.7 g; 10 mmol) at room temperature under vigorous stirring, and stirring was continued for 1 hour. A mixture of triethylamine (5.05 g; 50 mmol) and n-propanol (0.7 g; 11 mmol) was dropwise added therto under ice-cooling with stirring, and stirring was continued at room temperature overnight. The reaction mixture was diluted with ethyl acetate, washed with water twice, dried over anhydrous magnesium sulfate and concentrated under reduced pressure to give a crude oil. The oil was purified by column chromatography (eluent, n-hexane : ethyl acetate = 2 : 1) to give 1.4 g of 2-n-propoxy-2-(4-ethyl-2-methylthiazole-5-carboxamino)acetonitrile (Compound No. 7). m.p., 95 - 96° C. Yield, 52 %.

Example 5

To a solution of 2 (4-ethyl-2-methylthiazole-5-carboxamino)acetonitrile (2.1 g; 10 mmol) in ethyl acetate (40 ml), anhydrous magnesium sulfate (5 g) and bromine (3.2 g; 20 mmol) were added while stirring at room temperature. To the resultant suspension was added dropwise 47 % of hydrobromic acid (1.7 g; 10 mmol) at room temperature under vigorous stirring, and stirring was continued for 1 hour. A mixture of triethylamine (5.05 g; 50 mmol) and 1-butyn-3-ol (0.8 g; 11 mmol) was dropwise added thereto under ice-cooling with stirring, and stirring was continued at room temperature overnight. The reaction mixture was diluted with ethyl acetate, washed with water twice, dried over anhydrous magnesium sulfate and concentrated under reduced pressure to give a crude oil. The oil was purified by column chromatography (eluent, n-hexane : ethyl acetate = 2 : 1) to give 1.5 g of 2-(butynyl-3-oxy)-2-(4-ethyl-2-methylthiazole-5-carboxamino)acetonitrile (diastereo mixture) (Compound No. 10). m.p., 96 - 97° C. Yield, 54 %.

Example 6

To a solution of 2-(4-ethyl-2-methylthiazole-5-carboxamino)acetonitrile (2.1 g; 10 mmol) in ethyl acetate (40 ml), anhydrous magnesium sulfate (5 g) and bromine (3.2 g; 20 mmol) were added while stirring at room temperature. To the resultant suspension was added dropwise 47 % of hydrobromic acid (1.7 g; 10 mmol) at room temperature under vigorous stirring, and stirring was continued for 1 hour. A mixture of triethylamine (5.05 g; 50 mmol) and lactonitrile (0.8 g; 11 mmol) was dropwise added thereto under ice-cooling with stirring, and stirring was continued at room temperature overnight. The reaction mixture was diluted with ethyl acetate, washed with water twice, dried over anhydrous magnesium sulfate and concentrated under reduced pressure to give a crude oil. The oil was purified by column chromatography (eluent, n-hexane : ethyl acetate = 2 : 1) to give 1.1 g of 2-(2-cyanoethoxy)-2-(4-ethyl-2-methylthiazole-5-carboxamino)acetonitrile (diastereo mixture) (Compound No. 11). m.p., 136 - 138° C. Yield, 40 %.

Example 7

To a solution of 2-(4-ethyl-2-methylthiazole-5- carboxamino)acetonitrile (2.1 g; 10 mmol) in ethyl acetate (40 ml), anhydrous magnesium sulfate (5 g) and bromine (3.2 g; 20 mmol) were added while stirring at room temperature. To the resultant suspension was added dropwise 47 % of hydrobromic acid (1.7 g; 10 mmol) at room temperature under vigorous stirring, and stirring was continued for 1 hour. A mixture of triethylamine (5.05 g; 50 mmol) and ethanethiol (0.7 g; 11 mmol) was dropwise added thereto under ice-cooling with stirring, and stirring was continued at room temperature for 1 hour. The reaction mixture was diluted with ethyl acetate, washed with water twice, dried over anhydrous magnesium sulfate and concentrated under reduced pressure to give a crude oil. The oil was purified by column chromatography (eluent, n-hexane : ethyl acetate = 2 : 1) to give 2.0 g of 2-ethylthio-2-(4-ethyl-2-methylthiazole-5-carboxamino)acetonitrile (Compound No. 12) as a viscous oil. Yield, 74 %.

Example 8

To a solution of 2-(4-n-propyl-2-methylthiazole-5-carboxamino)acetonitrile (2.3 g; 10 mmol) in ethyl acetate (40 ml), anhydrous magnesium sulfate (5 g) and bromine (3.2 g; 20 mmol) were added while stirring at room temperature. To the resultant suspension was added dropwise 47 % of hydrobromic acid (1.7 g; 10 mmol) at room temperature under vigorous stirring, and stirring was continued for 1 hour. A mixture of triethylamine (5.1 g; 50 mmol) and ethanol (5 g; 108 mmol) was dropwise added thereto under ice-cooling with stirring, and stirring was continued at room temperature for 1 hour. The reaction mixture was diluted with ethyl acetate, washed with water twice, dried over anhydrous magnesium sulfate and concentrated under reduced pressure to give a crude oil. The oil was purified by column chromatography (eluent, n-hexane : ethyl acetate = 2 : 1) to give 1.6 g of 2-ethoxy-2-(4-n-propyl-2-methylthiazole-5-carboxamino)-acetonitrile (Compound No. 13). m.p., 123 - 124° C. Yield, 59 %.

Example 9

To a solution of 2-(4-isopropyl-2-methylthiazole-5-carboxamino)acetonitrile (2.3 g; 10 mmol) in ethyl acetate (40 ml), anhydrous magnesium sulfate (5 g) and bromine (3.2 g; 20 mmol) were added while stirring at room temperature. To the resultant suspension was added dropwise 47 % of hydrobromic acid (1.7 g; 10 mmol) at room temperature under vigorous stirring, and stirring was continued for 1 hour. A mixture of triethylamine (5.1 g; 50 mmol) and ethanol (5 g; 108 mmol) was dropwise added thereto under ice-cooling with stirring, and stirring was continued at room temperature for 1 hour. The reaction mixture was diluted with ethyl acetate, washed with water twice, dried over anhydrous magnesium sulfate and concentrated under reduced pressure to give a crude oil. The oil was purified by column chromatography (eluent, n-hexane : ethyl acetate = 2 : 1) to give 1.2 g of 2-ethoxy-2-(4-isopropyl-2-methylthiazole-5-carboxamino)-acetonitrile (Compound No. 14). m.p., 99 - 101 °C. Yield, 44 %.

Example 10 (Acetonitrile (IV))

To a solution of imidazole (13.6 g; 0.2 mol) in dry tetrahydrofuran (300 ml) was added dropwise thionyl chloride (5.9 g; 50 mmol) under ice-cooling with stirring, and stirring was continued at room temperature for 30 minutes. 2-Ethyl-4-methylthiazole-5-carboxylic acid (10.5 g; 50 mmol) was added thereto at room temperature, followed by stirring for 1 hour. Aminoacetonitrile hydrochloride (7 g; 75 mmol) was added to the reaction mixture, and triethylamine (7.6 g; 75 mmol) was dropwise added thereto at room temperature. After two hours, tetrahydrofuran was removed under reduced pressure, and the residue was diluted with water and extracted with ethyl acetate. The organic layer was washed with water, dried over anhydrous magnesium sulfate and concentrated under reduced pressure to give crude crystals, which were recrystallized from a mixture of ethyl acetate and n-hexane to give 6.6 g of 2-(4-ethyl-2-methylthiazole)acetonitrile as colorless crystals. m.p., 99 - 100 °C. Yield, 63 %.

In the same manner as above, the amide compounds (I) as shown in Table 1 and the acetonitriles (IV) as shown in Table 2 are obtained.

Table 1

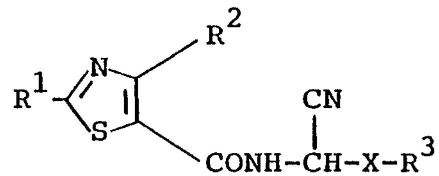

(I)

| Compuond No. | $R^1$ | $R^2$ | $XR^3$ | Physical property |
|---|---|---|---|---|
| 1 | $CH_3$ | $CH_3$ | $OCH_3$ | δ ppm ($CDCl_3$): 7.12 (d), 6.08 (d), 3.50 (s), 2.72 (s, 6H) |
| 2 | $CH_3$ | $CH_3$ | $OC_2H_5$ | δ ppm ($CDCl_3$): 7.52 (d), 6.13 (d), 3.75 (q), 2.68 (s, 6H) |
| 3 | $CH_3$ | $CH_3$ | $O-n-C_3H_7$ | δ ppm ($CDCl_3$): 7.40 (d), 6.15 (d), 3.62 (t), 2.65 (s, 6H), 1.9-1.5 (m), 0.92 (t) |
| 4 | $CH_3$ | $CH_3$ | $OCH(CH_3)C{\equiv}CH$ | δ ppm ($CDCl_3$): 6.90 (brd), 6.43, 6.40 (d), 6.55 (m), 2.68 (s, 6H), 1.53, 1.48 (d, 6H) |
| 5 | $CH_3$ | $C_2H_5$ | $OCH_3$ | m.p., 110 - 111°C |
| 6 | $CH_3$ | $C_2H_5$ | $OC_2H_5$ | m.p., 126 - 127°C |
| 7 | $CH_3$ | $C_2H_5$ | $O-n-C_3H_7$ | m.p., 95 - 96°C |

(Continued)

| Compuond No. | $R^1$ | $R^2$ | $XR^3$ | Physical property |
|---|---|---|---|---|
| 8 | $CH_3$ | $C_2H_5$ | O-iso-$C_3H_7$ | m.p., 121 - 122°C |
| 9 | $CH_3$ | $C_2H_5$ | $OCH_2C{\equiv}CH$ | m.p., 116 - 117°C |
| 10 | $CH_3$ | $C_2H_5$ | $OCH(CH_3)C{\equiv}CH$ | m.p., 96 - 97°C |
| 11 | $CH_3$ | $C_2H_5$ | $OCH(CH_3)CN$ | m.p., 135 - 136°C |
| 12 | $CH_3$ | $C_2H_5$ | $SC_2H_5$ | δ ppm ($CDCl_3$): 7.14 (d), 6.09 (d), 3.03 (q), 2.85 (q), 1.33 (t), 1.26 (t) |
| 13 | $CH_3$ | n-$C_3H_7$ | $OC_2H_5$ | m.p., 123 - 124°C |
| 14 | $CH_3$ | iso-$C_3H_7$ | $OC_2H_5$ | m.p., 99 - 100°C |
| 15 | $CH_3$ | $C_2H_5$ | $OCH_2CH_2F$ | m.p., 121 - 122°C |

Table 2

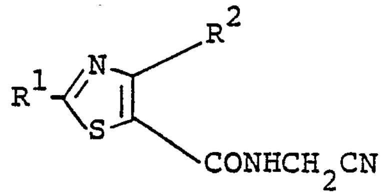

(IV)

| $R^1$ | $R^2$ | Physical property |
|---|---|---|
| $CH_3$ | $CH_3$ | m.p., 60 - 61°C |
| $C_2H_5$ | $CH_3$ | m.p., 76 - 77°C |
| $CH_3$ | $C_2H_5$ | m.p., 99 - 100°C |
| $CH_3$ | n-$C_3H_7$ | m.p., 91 - 92°C |
| $CH_3$ | iso-$C_3H_7$ | $n_D^{25}$ 1.5232 |

For the practical usage of the amide compounds (I) as fungicides, they may be applied as such or in preparation forms such as emulsifiable concentrates, wettable powders, suspensions, powders or granules. Such preparation forms can be formulated in a per se conventional manner, e.g. by mixing at least one of the amide compounds (I) with an appropriate solid or liquid carrier(s) or diluent(s) and, if necessary, one or more appropriate adjuvant(s) (e.g. surfactants, adherents, dispersants, stabilizers) for improving the dispersibility and other properties of the active ingredient.

Examples of the solid carriers or diluents are fine powders or granules of kaolin clay, attapulgite clay, bentonite, acid clay, pyrophyllite, talc, diatomaceous earth, calcite, corn rachis powders, walnut powders, urea, ammonium sulfate, synthetic hydrated silica, etc. As the liquid carrier or diluent, there may be exemplified aromatic hydrocarbons (e.g. xylene, methylnaphthalene), alcohols (e.g. isopropanol, ethylene glycol, cellosolve), ketones (e.g. acetone, cyclohexanone, isophorone), soybean oil, cotton seed oil, dimethylsulfoxide, acetonitrile, water, etc.

The surface active agent used for emulsification, dispersion or spreading may be any of the anionic and non-ionic type of agents. Examples of the surface active agent include anionic surfactants and non-ionic surfactans such as alkylsulfates, alkylarylsulfonates, dialkylsulfosuccinates, phosphates of polyoxyethylenealkylaryl ethers, condensates of naphthalenesulfonic acid and formalin, polyoxyethylene alkyl ethers, polyoxyethylene polyoxypropylene block copolymers, sorbitan fatty acid esters and polyoxyethylene sorbitan fatty acid esters. Examples of the auxiliary agents include ligninsulfonates, sodium alginate, polyvinyl alcohol, gum arabic, CMC (carboxymethyl cellulose), PAP (isopropyl acid phosphate), etc.

For the purpose of producing the fungicidal effect, the amide compounds (I) may be generally used in a range of about 0.01 to 50 grams, preferably about 0.05 to 10 grams, per are. In general, the concentration of the active ingredient in the fungicidal composition of the invention may be from about 0.01 to 99.9 % by weight, preferably from about 1 to 90 % by weight. For the practical use, the composition in the preparation form as above exemplified is diluted usually with water to make a concentration of about 0.0001 to 0.5 % by weight, preferably about 0.0005 to 0.2 % by weight, of the acitve ingredient, and then the resultant dilution is applied. When, however, the composition is formulated in dusts or granules, it is normally applied as such without dilution.

It is also notable that the amide compounds (I) may be used in admixture with other fungicides to enhance their fungicidal activity. Further, they may be applied in association with insecticides, miticides, nematocides, herbicides, plant-growth regulators, fertilizers, etc.

As stated above, the amide compounds (I) show appreciable fungicidal property. For instance, they exert preventive, curative and systemic effect against a wide variety of plant diseases caused by phytopathogenic fungi, of which typical examples are as follows: downy mildew of vegetables or radish (Peronospora brassicae), spinaches (Peronospora spinaciae), tobacco (Peronospora tabacina), cucumber

(Pseudoperonospora cubensis), grapes (Plasmopara viticola) or parsley (Plasmopara nivea), blight of apple, strawbery or ginseng (Phytophthora cactorum), tomato or cucumber (Phytophthora capsici), pineapple (Phytophthora cinnamomi), potato, tomato or eggplant (Phytophthora infestans) or tobacco, horse beans or onion (Phytophthora nicotianae var. nicotianae), damping-off of spinach (Phythium sp.) or cucumber (Pythium aphanidermatum), browning root rot of wheats (Phytium sp.), damping-off of tobacco seedlings (Pythium debaryanum), Phythium rot of soybeans (Phythium aphanidermatum, P. debaryanum, P. irregulare, P. myiotylum, P. ultimam), etc.

Some practical embodiments of the fungicidal composition according to the invention are illustratively shown in the following Formulation Examples wherein part(s) are by weight.

Formulation Example 1

Fifty parts of each of Compound Nos. 1 to 15, 3 parts of calcium ligninsulfonate, 2 parts of sodium laurylsulfonate and 45 parts of synthetic hydrated silica are mixed and thoroughly pulverized to obtain a wettable powder.

Formulation Example 2

Twenty-five parts of each of Compound Nos. 1 to 15, 3 parts of polyoxyethylene sorbitan monooleate, 3 parts of CMC and 69 parts of water are mixed and thoroughly pulverized until the particle size of the active ingredient becomes less than 5 microns to obtain a suspension.

Formulation Example 3

Two parts of each of Compound Nos. 1 to 15, 88 parts of kaolin clay and 10 parts of talc are mixed and thoroughly pulverized to obtain powders.

Formulation Example 4

Twenty parts of each of Compound Nos. 1 to 15, 14 parts of polyoxyethylene styrylphenyl ether, 6 parts of calcium dodecylbenzenesulfonate and 60 parts of xylene are mixed together to obtain an emulsifiable concentrate.

Formulation Example 5

Two parts of each of Compound Nos. 1 to 15, 1 part of synthetic hydrated silica, 2 parts of calcium ligninsulfonate, 30 parts of bentonite and 65 parts of kaolin clay are mixed and thoroughly pulverized with addition of water to obtain granules.

Typical test data indicating the excellent fungicidal activity of the amide compounds (I) are shown below. The assessment data are converted to percent disease control. 100 % means no infection; 0 % means that the plant are totally infected compared with the control plant.

Still, the compounds as shown in Table 3 were used for comparison:

Table 3

| Compound No. | Structure | Remarks |
| --- | --- | --- |
| A | 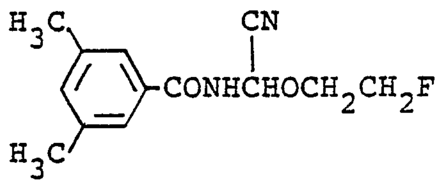 | JP-A-255759/87 |
| B | 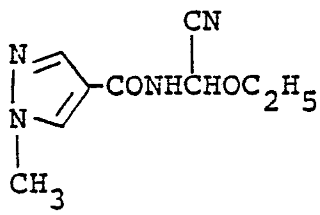 | EP-A-0268892 |

Test Example 1

Curative effect on late blight of tomato (Phytophthora infestans):-

A plastic pot was filled with sandy soil, and tomato (var: Ponte Rosa) were sowed therein, followed by cultivation in a greenhouse for 20 days. A spore suspension of Phytophthora infestans was inoculated by spreading over the seedlings at the 2 to 3 leaf-stage of the test plants, which were then allowed to stand at 20°C overnight under a humid condition. The test compound formulated in a wettable powder according to Formulation Example 1 and diluted with water to a prescribed concentration was thoroughly sprayed over the test plants, which were further grown for 5 days under illumination and observed. The results are shown in Table 4.

Test Example 2

Curative effect on downy mildew of grapes (Plasmopara viticola):-

A plastic pot was filled with sandy soil, and seeds of grapes (var: Bailey-A) were sowed therein, followed by cultivation in a greenhouse for 50 days. A spore suspension of Plasmopara viticola was inoculated by spreading over the seedlings at the 6 to 7 leaf-stage of the test plants, which were then allowed to stand at 20°C overnight under a humid condition. The test compound formulated in a wettable powder according to Formulation Example 1 and diluted with water to a prescribed concentration was thoroughly sprayed over the test plants, which were further grown for 8 days under illumination and observed. The results are shown in Table 4.

Test Example 3

Curative effect on downy mildew of cucumber (Pseudoperonospora cubensis):-

A plastic pot was filled with sandy soil, and seeds of cucumber (var: Sagamihanjiro) were sowed therein, followed by cultivation in a greenhouse for 14 days. A spore suspension of Pseudoperonospora cubensis was inoculated by spreading over the seedlings of the test plants, which were then allowed to

stand at 20°C overnight under a humid condition. The test compound formulated in a wettable powder according to Formulation Example 1 and diluted with water to a prescribed concentration was thoroughly sprayed over the test plants, which were further grown for 8 days under illumination and observed. The results are shown in Table 4.

Table 4

| Compound No. | Concentration (ppm) | Curative effect | | |
|---|---|---|---|---|
| | | Tomato | Grape | Cucumber |
| 1 | 200 | 100 | 100 | 100 |
| | 100 | 95 | 90 | - |
| 2 | 200 | 100 | 100 | 100 |
| | 100 | 95 | 100 | 95 |
| 3 | 200 | 100 | 100 | 100 |
| | 100 | 95 | 95 | 90 |
| 4 | 200 | 100 | 100 | 100 |
| | 100 | 90 | - | - |
| 5 | 200 | 100 | 100 | 100 |
| | 100 | 95 | 90 | - |
| 6 | 200 | 100 | 100 | 100 |
| | 100 | 100 | 100 | - |
| 7 | 200 | 100 | 100 | 100 |
| | 100 | 100 | 90 | 90 |
| 8 | 200 | 100 | 100 | 100 |
| | 100 | 95 | 90 | 95 |
| 9 | 200 | 100 | 100 | 100 |
| | 100 | 90 | 95 | 100 |
| 10 | 200 | 100 | 100 | 100 |
| | 100 | 95 | 90 | 95 |
| 11 | 200 | 100 | 100 | 100 |
| | 100 | 100 | 100 | 90 |
| 12 | 200 | 100 | 100 | 100 |
| | 100 | 95 | 90 | 90 |
| 13 | 200 | 95 | 90 | 95 |
| | 100 | 90 | - | 90 |
| 14 | 200 | 100 | 100 | 100 |
| | 100 | 100 | 90 | 100 |
| B | 200 | 0 | 0 | 0 |
| | 100 | - | 0 | 0 |

Test Example 4

Phytotoxicity:-

Over the seedlings of grapes (var: Bailey-A), tomato (var: Ponte Rosa) and cucumber (var: Sagamihanjiro), the test compound formulated in a wettable powder according to Formulation Example 1 and diluted with water to a prescribed concentration was sprayed, and the test plants were further grown in a greenhouse for 2 weeks and subjected to observation for the phytotoxicity to the test plants. The phytotoxicity was visually observed and rated as 0, 1, 2, 3, 4 or 5 depending upon the degree of damage wherein 5 indicates no growth of the plants due to severe phytotoxicity whereas 0 indicates no phytotoxicity. The results are shown in Table 5.

Table 5

| Compound No. | Concentration (ppm) | Phytotoxicity | | |
|---|---|---|---|---|
| | | Tomato | Grape | Cucumber |
| 1 | 500 | 0 | 0 | 0 |
| 2 | 500 | 0 | 0 | 0 |
| 3 | 500 | 0 | 0 | 0 |
| 4 | 500 | 0 | 0 | 0 |
| 5 | 500 | 0 | 0 | 0 |
| 6 | 500 | 0 | 0 | 0 |
| 7 | 500 | 0 | 0 | 0 |
| 8 | 500 | 0 | 0 | 0 |
| 9 | 500 | 0 | 0 | 0 |
| 10 | 500 | 0 | 0 | 0 |
| 11 | 500 | 0 | 0 | 0 |
| 12 | 500 | 0 | 0 | 0 |
| 13 | 500 | 0 | 0 | 0 |
| 14 | 500 | 0 | 0 | 0 |
| 15 | 500 | 0 | 0 | 0 |
| A | 500 | 3 | 0 | 3 |

## Claims

1. An amide compound of the formula:

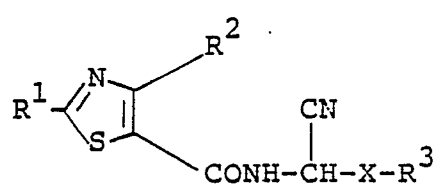

wherein $R^1$ and $R^2$ are, the same or different, each a a lower alkyl group, $R^3$ is a lower alkyl group, a halo(lower)alkyl group, a cyano(lower)alkyl group or a lower alkynyl group and X is an oxygen atom or a sulfur atom.

2. The amide compound according to claim 1, which is in an optically active form.

3. The amide compound according to claim 1, wherein $R^1$ is a methyl group, $R^2$ is a methyl group or an ethyl group and $R^3$ is a $C_1$-$C_3$ alkyl group, a halo($C_2$-$C_3$)alkyl group, a cyano($C_1$-$C_3$)alkyl group or a $C_3$-$C_5$ alkynyl group.

4. The amide compound according to claim 1, wherein $R^1$ is a methyl group, $R^2$ is a methyl group or an ethyl group and $R^3$ is a $C_1$-$C_3$ alkyl group.

5. The amide compound according to claim 1, wherein $R^1$ is a methyl group, $R^2$ is an ethyl group and $R^3$ is an ethyl group.

6. A process for producing an amide compound of the formula:

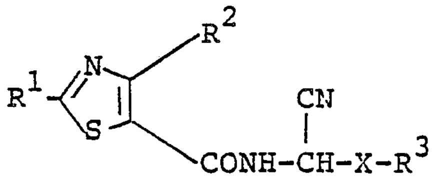

wherein $R^1$ and $R^2$ are, the same or different, each a a lower alkyl group, $R^3$ is a lower alkyl group, a halo-(lower)alkyl group, a cyano(lower)alkyl group or a lower alkynyl group and X is an oxygen atom or a sulfur atom, which comprises reacting a haloacetonitrile of the formula:

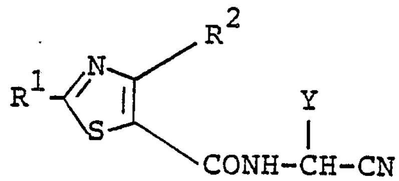

wherein $R^1$ and $R^2$ are each as defined above and Y is a halogen atom with a compound of the formula:
$R^3$-X-H
wherein $R^3$ and X are each as defined above.

7. The process according to claim 6, wherein the haloacetonitrile compound is the one prepared by reacting an acetonitrile of the formula:

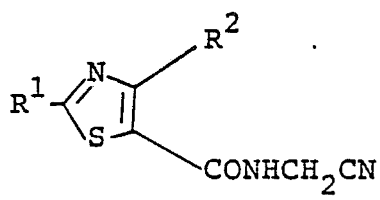

wherein $R^1$ and $R^2$ are each as defined above with a haloge nating agent.

8. A fungicidal composition which comprises as an active ingredient a fungicidally effective amount of an amide compound of the formula:

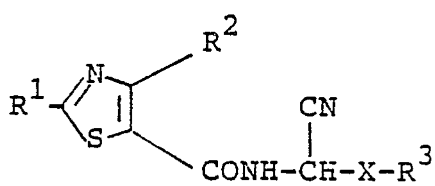

wherein $R^1$ and $R^2$ are, the same or different, each a a lower alkyl group, $R^3$ is a lower alkyl group, a halo-(lower)alkyl group, a cyano(lower)alkyl group or a lower alkynyl group and X is an oxygen atom or a sulfur atom, and an inert carrier or diluent.

9. A method for controlling phytopathogenic fungi, which comprises applying a fungicidally effective amount of an amide compound of the formula:

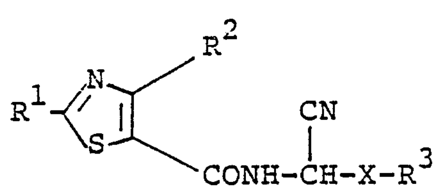

wherein $R^1$ and $R^2$ are, the same or different, each a a lower alkyl group, $R^3$ is a lower alkyl group, a halo-

(lower)alkyl group, a cyano(lower)alkyl group or a lower alkynyl group and X is an oxygen atom or a sulfur atom to phytopathogenic fungi.

10. An acetonitrile of the formula:

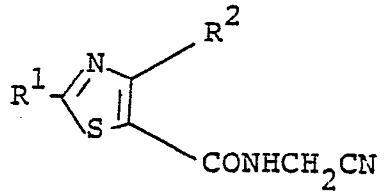

wherein $R^1$ and $R^2$ are, the same or different, each a $C_1$-$C_3$ alkyl group.

11. A haloacetonitrile of the formula:

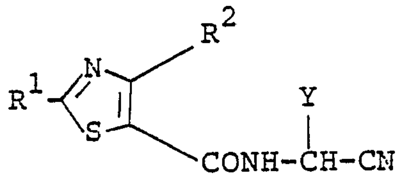

wherein $R^1$ and $R^2$ are, the same or different, each a $C_1$-$C_3$ alkyl group and Y is a halogen atom.

12. A process for producing an acetonitrile of the formula:

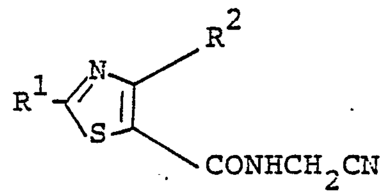

wherein $R^1$ and $R^2$ are, the same or different, each a $C_1$-$C_3$ alkyl group, which comprises reacting a thiazolecarboxylic acid halide of the formula:

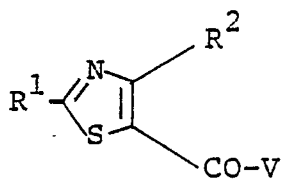

wherein $R^1$ and $R^2$ are each as defined above and V is a halogen atom with aminoacetonitrile of the formula:
$H_2NCH_2CN$
or its salt.

13. A process for producing an acetonitrile of the formula:

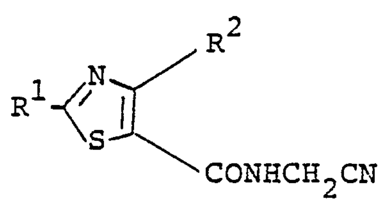

wherein $R^1$ and $R^2$ are, the same or different, each a $C_1$-$C_3$ alkyl group, which comprises reacting a thiazolecarboxylic acid of the formula:

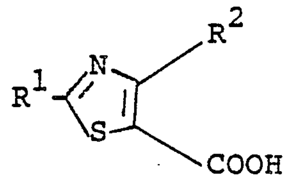

wherein $R^1$ and $R^2$ are each as defined above with a diimidazole of the formula:

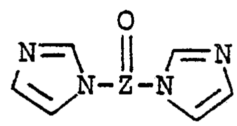

wherein Z is a carbon atom or a sulfur atom to give.an acyl imidazole of the formula:

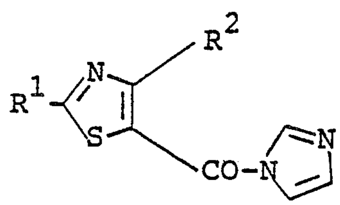

wherein $R^1$ and $R^2$ are each as defined above and reacting the acylimidazole with aminoacetonitrile of the formula:
$H_2NCH_2CN$
or its salt.

14. A process for producing an acetonitrile of the formula:

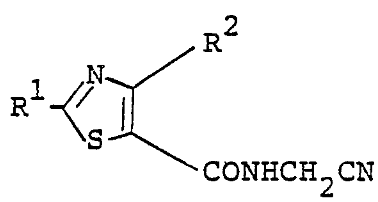

wherein $R^1$ and $R^2$ are, the same or different, each a $C_1$-$C_3$ alkyl group, which comprises reacting an acylacetonitrile of the formula:
$R^2$-$COCH_2CONHCH_2CN$
wherein $R^2$ is as defined above with a thioacetamide compound of the formula:
S
||
$R^1$- C $NH_2$
wherein $R^1$ is as defined above in the presence of a halogenating agent.

15. Use of an amide compound of the formula:

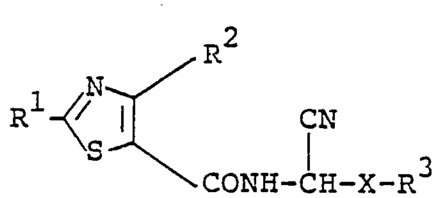

wherein $R^1$ and $R^2$ are, the same or different, each a a lower alkyl group, $R^3$ is a lower alkyl group, a halo(lower)alkyl group, a cyano(lower)alkyl group or a lower alkynyl group and X is an oxygen atom or a sulfur atom as a fungicide.

Claims for the following Contracting State : ES

1. A process for producing an amide compound of the formula:

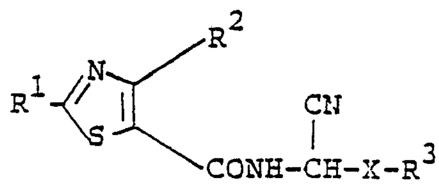

wherein $R^1$ and $R^2$ are, the same or different, each a a lower alkyl group, $R^3$ is a lower alkyl group, a halo(lower)alkyl group, a cyano(lower)alkyl group or a lower alkynyl group and X is an oxygen atom or a sulfur atom, which comprises reacting a haloacetonitrile of the formula:

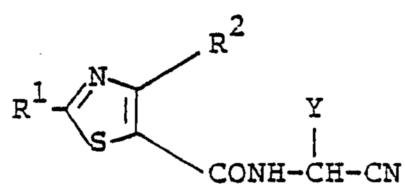

wherein $R^1$ and $R^2$ are each as defined above and Y is a halogen atom with a compound of the formula:
$R^3$-X-H
wherein $R^3$ and X are each as defined above.

2. The process according to claim 1, wherein the compound prepared is in an optically active form.

3. The process according to claim 1, wherein in the compound prepared $R^1$ is a methyl group, $R^2$ is a methyl group or an ethyl group and $R^3$ is a $C_1$-$C_3$alkyl group, a halo ($C_2$-$C_3$)alkyl group, a cyano ($C_1$-$C_3$)-alkyl group or a $C_3$-$C_5$ alkynyl group.

4. The process according to claim 1, wherein in the compound prepared $R^1$ is a methyl group, $R^2$ is a methyl group or an ethyl group and $R^3$ is a $C_1$-$C_3$ alkyl group.

5. The process according to claim 1, wherein in the compound prepared $R^1$ is a methyl group, $R^2$ is an ethyl group and $R^3$ is an ethyl group.

6. The process according to claim 1, wherein the haloacetonitrile compound is the one prepared by reacting an acetonitrile of the formula:

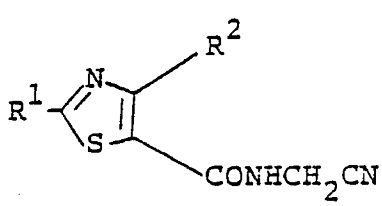

wherein $R^1$ and $R^2$ are each as defined above with a halogenating agent.

7. A fungicidal composition which comprises as an active ingredient a fungicidally effective amount of an amide compound of the formula:

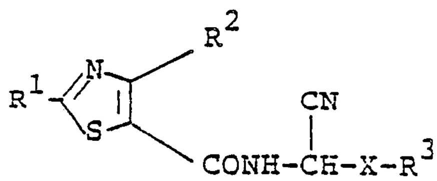

wherein $R^1$ and $R^2$ are, the same or different, each a a lower alkyl group, $R^3$ is a lower alkyl group, a halo(lower)alkyl group, a cyano(lower)alkyl group or a lower alkynyl group and X is an oxygen atom or a sulfur atom, and an inert carrier or diluent.

8. A method for controlling phytopathogenic fungi, which comprises applying a fungicidally effective amount of an amide compound of the formula:

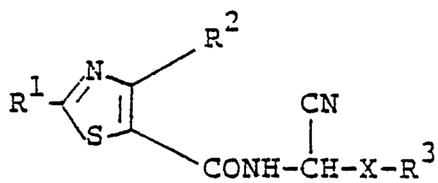

wherein $R^1$ and $R^2$ are, the same or different, each a a lower alkyl group, $R^3$ is a lower alkyl group, a halo(lower)alkyl group, a cyano(lower)alkyl group or a lower alkynyl group and X is an oxygen atom or a sulfur atom to phytopathogenic fungi.

9. A process for producing a haloacetonitrile of the formula:

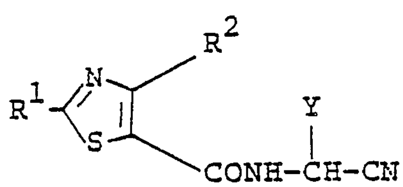

wherein $R^1$ and $R^2$ are, the same or different, each a $C_1$-$C_3$ alkyl group and Y is a halogen atom, which comprises reacting an acetonitrile of the formula:

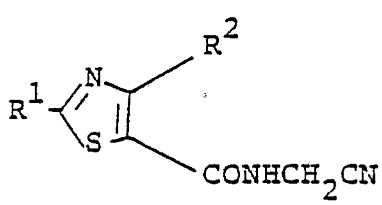

wherein $R^1$ and $R^2$ are, the same or different, each a $C_1$-$C_3$ alkyl group, with a halogenating agent.

10. A process for producing an acetonitrile of the formula:

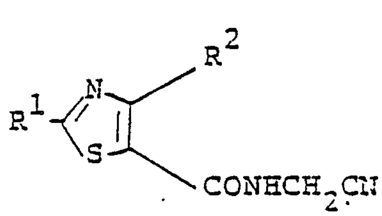

wherein $R^1$ and $R^2$ are, the same or different, each a $C_1$-$C_3$ alkyl group, which comprises reacting a thiazolecarboxylic acid halide of the formula:

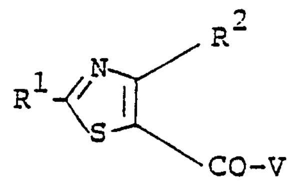

wherein $R^1$ and $R^2$ are each as defined above and V is a halogen atom with aminoacetonitrile of the formula:
$H_2NCH_2CN$
or its salt.

11. A process for producing an acetonitrile of the formula:

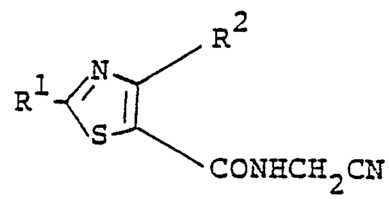

wherein $R^1$ and $R^2$ are, the same or different, each a $C_1$-$C_3$ alkyl group, which comprises reacting a thiazolecarboxylic acid of the formula:

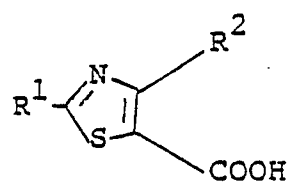

wherein $R^1$ and $R^2$ are each as defined above with a diimidazole of the formula:

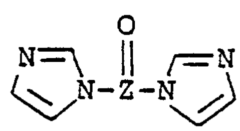

wherein Z is a carbon atom or a sulfur atom to give an acyl imidazole of the formula:

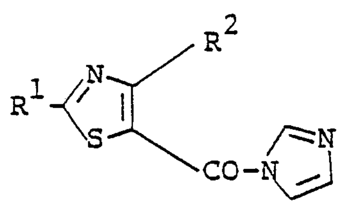

wherein $R^1$ and $R^2$ are each as defined above and reacting the acylimidazole with aminoacetonitrile of the formula:
$H_2NCH_2CN$
or its salt.

12. A process for producing an acetonitrile of the formula:

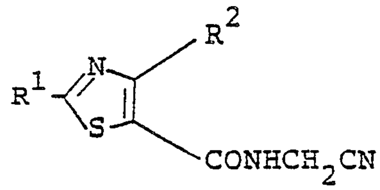

wherein $R^1$ and $R^2$ are, the same or different, each a $C_1$-$C_3$ alkyl group, which comprises reacting an acylacetonitrile of the formula:

$R^2$-$COCH_2CONHCH_2CN$

wherein $R^2$ is as defined above with a thioacetamide compound of the formula:

S
||
$R^1$- C $NH_2$

wherein $R^1$ is as defined above in the presence of a halogenating agent.

13. Use of an amide compound of the formula:

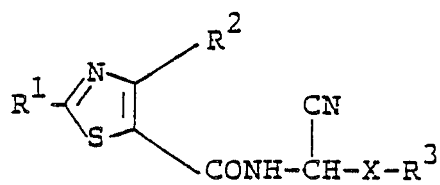

wherein $R^1$ and $R^2$ are, the same or different, each a a lower alkyl group, $R^3$ is a lower alkyl group, a halo-(lower)alkyl group, a cyano(lower)alkyl group or a lower alkynyl group and X is an oxygen atom or a sulfur atom as a fungicide.